# EUROPÄISCHE PATENTSCHRIFT

(11) **EP 1 972 308 B1**
(45) Veröffentlichungstag und Bekanntmachung des Hinweises auf die Patenterteilung: **03.11.2010**
(21) Anmeldenummer: 08151509.0
(22) Anmeldetag: 15.02.2008
(51) Int. Cl.: A61F 2/36

(54) **Gelenkkugel oder Kappenimplantat für ein künstliches Hüftgelenk**
Ball socket or cap implant for an artificial hip joint.
Rotule ou implant à capuchon pour une prothèse de hanche artificielle.

(30) Priorität: 21.03.2007 DE 102007014265
(43) Veröffentlichungstag der Anmeldung: 24.09.2008
(73) Patentinhaber: ESKA Implants GmbH & Co. KG, 23556 Lübeck (DE)
(72) Erfinder: Grundei, Hans, 23558, Lübeck (DE)
(74) Vertreter: Hughes, Andrea Michelle

(56) Entgegenhaltungen:
- WO-A-03/044383
- DE-B3- 10 318 374
- DE-C1- 19 750 121
- US-A- 5 879 406
- US-A- 5 916 269
- US-A- 6 045 581
- US-A1- 2005 182 494

## Beschreibung

Die vorliegende Erfindung betrifft eine Gelenkkugel bzw. ein Kappenimplantat für ein künstliches Hüftgelenk, die bzw. das dazu geeignet ist, in einer künstlichen Hüftgelenkspfanne eine Rotations- und Schwenkbewegung auszuführen, wobei zwischen der Gelenkkugel bzw. dem Kappenimplantat und der künstlichen Hüftgelenkspfanne ein Spalt für einen natürlichen Flüssigkeitsfilm, die Synovia, ausgebildet ist und in Bereichen ihrer Oberfläche regelmäßige Vertiefungen ohne Verbindungskanäle untereinander eingelassen sind.

Eine derartige Gelenkkugel ist bekannt aus der DE 103 18 374 B3.

Ein grundsätzliches Problem bei künstlichen Hüftgelenken ist die Reibung zwischen den Gelenkteilen Gelenkkugel und Hüftgelenkspfannen, die ihrerseits zu Verschleißerscheinungen führt, die medizinische Komplikationen nach sich ziehen können.

Der Erfindung der zuvor genannten Druckschrift lag nun die Bemühung zugrunde, eine Gelenkkugel bzw. -kappe so weiter zu bilden, dass einerseits eine Reibungsminimierung und damit eine Verschleißminimierung erreicht wird bei erheblich reduzierten Herstellungskosten. Hierzu ist vorgeschlagen worden, dass bei einer Gelenkkugel mit wenigstens drei zirkulär um die Polachse verlaufenden Nuten die Nuten ohne Verbindungskanäle untereinander in die Kugeloberfläche eingebracht sind. Hierdurch wird zunächst eine Kontaktflächenminimierung erzielt, da im Bereich der Nuten kein direkter Kontakt zwischen der Gelenkkugel und der künstlichen Hüftgelenkspfanne besteht. Die zirkulär verlaufenden Nuten sind nach der Implantation gefüllt mit Synovia. Die Nuten üben dabei keinen abpuffernden Effekt, sondern vielmehr eine tragende Rolle aus. Darüber hinaus entsteht durch die flüssigkeitsgefüllten Nuten ein Vakuum zwischen der Gelenkkugel und der Hüftgelenkspfanne, welches seinerseits ein deutliches Luxationshemmnis darstellt.

Wenn dieser Ansatz auch schon recht viel versprechend war, so ist zu bemerken, dass der Schmiereffekt der Synovia in dem Spalt zwischen der Gelenkkugel und der Hüftgelenkspfanne nicht optimal ausgenutzt wird zum Schmieren der Gelenkkugel gegenüber der Hüftgelenkspfanne einerseits aber auch zum Abtransport etwaiger Abriebspartikel.

Vor diesem Hintergrund ist es die Aufgabe der vorliegenden Erfindung ein Gelenkkugel bzw. -kappe der eingangs genannten Art vorzuschlagen, bei welcher der Schmier- und Reinigungseffekt gegenüber dem Stand der Technik deutlich erhöht ist.

Erfindungsgemäß wird zur Lösung dieser Aufgabe vorgeschlagen,
- dass die Oberfläche der Gelenkkugel bzw. -kappe im Polbereich ohne Vertiefungen glatt ist,
- dass in einem Zwischenbereich zwischen dem glatten Polbereich und einem Bereich oberhalb ihres Äquators ein Bereich vorgesehen ist, in dem Vertiefungen eingelassen sind mit Öffnungsweiten zwischen 1,0 mm bis 3,0 mm,
- dass in Richtung ihres anderen Pols weitere Vertiefungen eingelassen sind, deren Öffnungsweiten stetig kleiner werden, je näher sie an der Basiskante liegen und Öffnungsweiten zwischen 0,5 mm und 1,0 mm aufweisen.

Vom Zwischenbereich unterhalb des Polbereichs hin zu ihrer Basiskante ist die Oberfläche der Gelenkkugel bzw. -kappe also graduiert strukturiert in dem Sinne, dass ausgehend vom Zwischenbereich eine zunächst etwas gröbere Struktur in Form der eingelassenen Vertiefungen vorgesehen ist. Geht man nun weiter über den Äquator hinaus, so schließt sich dem Zwischenbereich unterhalb des Äquators ein Bereich an, welcher mit Vertiefungen kleinerer Dimension versehen ist, und zwar vom Anschluss an den Zwischenbereich bis hin zur Basiskante kleiner werdend.

Zusätzlich kann die Oberfläche im Bereich zwischen Äquator und Basiskante mit Vertiefungen mit Weiten zwischen 50 µm und 250 µm strukturiert sein, die beispielsweise durch Laserstrahlen erzeugt sind.

Die Oberfläche der erfindungsgemäßen Gelenkkugel ist also im Wesentlichen in drei Bereiche aufgeteilt. Jeder dieser Bereiche ist den ihm übertragenen Funktionen angepasst.

Der glatte Polbereich ist die Ablastzone der Gelenkkugel, da in diesen beim Gehen die größten Kräfte eingeleitet werden.

Der Zwischenbereich erfüllt die Funktion eines Reservoirs für die Synovia und baut gleichzeitig eine Pufferfunktion auf. Dies gelingt daher, weil die Vertiefungen keinerlei Verbindungskanäle untereinander aufweisen, so dass sich der in den einzelnen Vertiefungen aufbauende Druck zu einem Druckpuffer aufbaut.

Der Bereich unterhalb des Äquators dient nicht nur zum Schmieren des Gelenkes, sondern vor allem auch zur Reinigung, das heißt Abtransport etwaiger Abriebs- oder sonstiger Partikel. Die diskreten makroskopischen Vertiefungen in diesem Bereich mit den Weiten oberhalb von 0,5 mm dienen in erster Linie der Schmierung des Gelenkes. Die Mikrostrukturen mit Weiten zwischen 50 µm und 250 µm dienen in erster Linie dem Abtransport der erwähnten Partikel. Dies funktioniert analog zu dem bekannten Lotuseffekt.

Gemäß einer vorteilhaften Weiterbildung weisen die makroskopischen Vertiefungen eine runde Öffnung auf. Durch diese Gestaltung lassen sich bestimmte Flächendrücke der Synovia in den Vertiefungen aufbauen.

Gemäß einer noch weiteren vorteilhaften Ausführungsform ist vorgesehen, dass die Vertiefungen im Querschnitt kalottenförmig ausgebildet sind. Hierdurch bieten sie einerseits ein genügend großes Reservoir für die Synovia und andererseits die Möglichkeit einer guten Umspülung und Austausch der in den Vertiefungen gespeicherten Synovia gegen nachströmende Synovia.

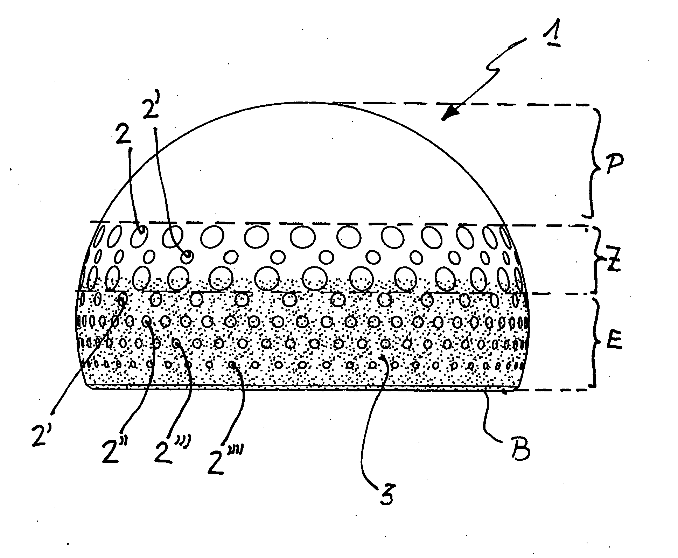

Eine noch weitere vorteilhafte Ausführungsform sieht vor, dass im Zwischenbereich oberhalb des Äquators Vertiefungen mit alternierenden Öffnungsweiten zwischen 1,0 mm und 3,0 mm in die Oberfläche eingelassen sind. Hierdurch lassen sich gezielt aufzubauende Drücke einstellen. Die Anordnung kann vorsehen, dass die Vertiefungen mit den größeren Öffnungsweiten quasi wie ein Kranz auf der Oberfläche umläuft. Diesem ersten Kranz könnte sich dann ein zweiter Kranz anschließen mit Vertiefungen mit kleineren Öffnungsweiten, der dann wiederum gefolgt werden kann von einem dritten Kranz wiederum mit Vertiefungen mit den größeren Öffnungsweiten. Dieser exakt geometrischen Anordnung bedarf es jedoch nicht zwingend. Denkbar wären auch Anordnungen von Vertiefungen ohne strenge geometrische Ausrichtung.
Die Erfindung wird anhand eines Ausführungsbeispiels gemäß der einzigen Zeichnungsfigur näher erläutert.

Diese zeigt schematisch eine Seitenansicht der erfindungsgemäß ausgebildeten Gelenkkugel bzw. -kappe.

Wie zu erkennen ist, ist die Gelenkkugel 1 kalottenförmig ausgebildet. Im Polbereich P ist die Oberfläche vollkommen glatt. Dieser Abschnitt bildet den in erster Linie Last aufnehmenden Teil der Gelenkkugel. Die Oberfläche der Gelenkkugel 1 zeichnet sich im weiteren Verlauf durch eine graduierte Strukturierung aus.

Dem Polbereich P schließt sich äquatorwärts ein Zwischenbereich Z an. Dieser ist durch eine Makrostruktur gekennzeichnet, gebildet aus Vertiefungen 2 und 2'. Wie zu erkennen ist, wechseln sich die größeren Vertiefungen 2 mit den kleineren Vertiefungen 2' ab, sie sind alternierend angeordnet. Die Vertiefungen 2 und 2' dienen in erster Linie zur Aufnahme eines Reservoirs für die Synovia.

Dem Zwischenbereich Z schließt sich etwa unterhalb des Äquators ein Bereich E an. Dieser ist zum einen gekennzeichnet durch noch makroskopische Vertiefungen mit Öffnungsweiten zwischen 0,5 mm und 1,2 mm. Diese dienen auch noch als Reservoir für die Synovia. Je kleiner sie allerdings werden, desto zunehmender dienen sie der Reinigung der Synovia. Unterlegt ist diese Makrostruktur in dem Bereich E durch eine Oberfläche 3, die in der Zeichnungsfigur durch die graue Markierung angedeutet und durch Laserstrahlen hergestellt ist . Diese mikroskopische Oberflächenstruktur dient in erster Linie der Reinigung des transportierten Liquids.

Durch den dargestellten Aufbau ergibt sich eine optimale Funktionsverteilung der verschiedenen Bereiche der Gelenkkugel. So ist sichergestellt, dass der glatte Polbereich stets mit Synovia benetzt ist. Andernfalls würde dort ein Abrieb katastrophalen Ausmaßes stattfinden. Sollten dennoch einmal Partikel sich in der Synovia befinden, so werden diese durch das Design der Oberflächen mit der graduierten Strukturierung nach außen befördert.

## Patentansprüche

1. Gelenkkugel oder -kappe (1) für ein künstliches Hüftgelenk, die dazu geeignet ist, in einer künstlichen Hüftgelenkspfanne eine Rotations- und Schwenkbewegung auszuführen, wobei zwischen ihr und der künstlichen Hüftgelenkspfanne ein Spalt für einen natürlichen Flüssigkeitsfilm ausgebildet ist, und in Bereichen ihrer Oberfläche regelmäßige Vertiefungen ohne Verbindungskanäle untereinander eingelassen sind,
**dadurch gekennzeichnet,**
- **dass** ihre Oberfläche im Polbereich (P) ohne Vertiefungen glatt ist,
- **dass** in einem Zwischenbereich (Z) zwischen dem glatten Polbereich (P) und etwa ihrem Äquator ein Bereich vorgesehen ist, in dem Vertiefungen (2, 2') eingelassen sind mit Öffnungsweiten zwischen 1,0 bis 3,0 mm,
- **dass** an dem Zwischenbereich (Z) in Richtung ihrer Basiskante (B) anschließend weitere Vertiefungen (2', 2", 2"', 2"") eingelassen sind, deren Öffnungsweiten stetig kleiner werden, je näher sie an der Basiskante (B) liegen und Öffnungsweiten zwischen 0,5 und 1,0 mm aufweisen.

2. Gelenkkugel (1) nach Anspruch 1, **dadurch gekennzeichnet, dass** die Oberfläche im Bereich zwischen Äquator und Basiskante (B) zusätzlich mit Vertiefungen (3) mit Weiten zwischen 50 und 250 µm versehen ist.

3. Gelenkkugel (1) nach Anspruch 1 oder 2, **dadurch gekennzeichnet, dass** die Vertiefungen (2, 2', 2", 2"', 2"") jeweils eine runde Öffnung aufweisen.

4. Gelenkkugel (1) nach einem der Ansprüche 1 bis 3, **dadurch gekennzeichnet, dass** die Vertiefungen (2, 2', 2", 2"', 2"") im Querschnitt kalottenförmig ausgebildet sind.

5. Gelenkkugel (1) nach einem der Ansprüche 1 bis 4, **dadurch gekennzeichnet, dass** im Zwischenbereich (Z) Vertiefungen (2, 2') mit alternierenden Öffnungsweiten zwischen 1,0 und 3,0 mm in die Oberfläche eingelassen sind.

## Claims

1. Joint head or joint cap (1) for an artificial hip joint, which is suitable for executing a rotational and pivoting movement in an artificial hip joint socket, wherein a gap for a natural film of liquid is formed between it and the artificial hip joint socket, and regular depressions without connecting channels to one another are admitted in regions of its surface, **characterised in that**
- its surface in the pole region (P) is smooth without depressions,
- **in that** in an intermediate region (Z) between the smooth pole region (P) and approximately its equator, a region is provided in which depressions (2, 2') are admitted having opening widths between 1.0 to 3.0 mm,
- **in that** adjoining the intermediate region (Z) in the direction of its base edge (B), further depressions (2', 2", 2"', 2"") are admitted, the opening widths of which become constantly smaller the closer they lie to the base edge (B) and have opening widths between 0.5 and 1.0 mm.

2. Joint head (1) according to claim 1, **characterised in that** the surface in the region between equator and base edge (B) is additionally provided with depressions (3) having widths between 50 and 250 µm.

3. Joint head (1) according to claim 1 or 2, **characterised in that** the depressions (2, 2', 2", 2"', 2"") have in each case a round opening.

4. Joint head (1) according to one of claims 1 to 3, **characterised in that** the depressions (2, 2', 2", 2"', 2"") are designed to be dome-shaped in cross-section.

5. Joint head (1) according to one of claims 1 to 4, **characterised in that** in the intermediate region (Z), depressions (2, 2') having alternating opening widths between 1.0 and 3.0 mm are admitted into the surface.

## Revendications

1. Rotule ou calotte sphérique (1) pour une articulation de hanche artificielle, apte à effectuer un mouvement de rotation et de pivotement dans une cavité cotyloïde artificielle, un interstice pour un film liquide naturel étant formé entre elle et la cavité cotyloïde artificielle, et des évidements réguliers sans canaux de jonction étant ménagés les uns sous les autres dans des zones de sa surface,
**caractérisée**
- **en ce que** sa surface est lisse et sans évidements dans la zone polaire (P),
- **en ce que**, dans une zone intermédiaire (Z) entre la zone polaire lisse (P) et sensiblement son équateur, une zone est prévue où des évidements (2, 2') sont ménagés avec des largeurs d'ouverture entre 1,0 et 3,0 mm,
- **en ce que**, adjacents à la zone intermédiaire (Z) dans la direction de son bord de base (B), sont ménagés d'autres évidements (2', 2", 2"', 2"") dont les largeurs d'ouverture sont progressivement réduites à mesure qu'ils se rapprochent du bord de base (B), et dont les largeurs d'ouverture sont comprises entre 0,5 et 1,0 mm.

2. Rotule (1) selon la revendication 1, **caractérisée en ce que**, dans la zone entre l'équateur et le bord de base (B), la surface est en outre pourvue d'évidements (3) de largeurs comprises entre 50 et 250 µm.

3. Rotule (1) selon la revendication 1 ou la revendication 2, **caractérisée en ce que** les évidements (2, 2', 2", 2"', 2"") présentent chacun une ouverture ronde.

4. Rotule (1) selon l'une des revendications 1 à 3, **caractérisée en ce que** les évidements (2, 2', 2", 2"', 2"") sont prévus avec une section en forme de calotte.

5. Rotule (1) selon l'une des revendications 1 à 4, **caractérisée en ce que**, dans la zone intermédiaire (Z), des évidements (2, 2') avec des largeurs d'ouverture alternées entre 1,0 et 3,0 mm sont ménagés à la surface.
